# EUROPEAN PATENT APPLICATION

(11) **EP 4 656 721 A1**
(43) Date of publication of application: **03.12.2025**
(21) Application number: 24382575.9
(22) Date of filing: 29.05.2024
(51) Int. Cl.: C12N 9/90, C12N 9/96

(54) **HSP60 PROTEINS OBTAINED BY MEANS OF ANCESTRAL SEQUENCE RECONSTRUCTION (ASR)**

(71) Applicant: Instituto Nacional De Tecnica Aeroespacial "Esteban Terradas", 28850 Torrejón de Ardoz (ES); Consejo Superior De Investigaciones Científicas - CSIC, 28006 Madrid (ES)
(72) Inventor: PARRO GARCÍA, Víctor, 28850 Torrejón de Ardoz (ES); DOS SANTOS, Rita Sofía, 28850 Torrejón de Ardoz (ES); MORENO PAZ, Mercedes, 28850 Torrejón de Ardoz (ES); VALPUESTA, José María, 28006 Madrid (ES); CUÉLLAR, Jorge, 28006 Madrid (ES); MAESTRO LÓPEZ, Moisés, 28006 Madrid (ES)
(74) Representative: TRBL Intellectual Property

(57) **Abstract**

The present invention refers to an HSP60 protein, wherein the HSP60 protein has an amino acid sequence that has at least an 85% sequence identity with any one of SEQ ID NOS: 1-4, and to nucleotide sequences encoding the same. The invention also refers to compositions comprising an Hsp60 protein and their use as a detergent, or as a juice clarifying agent. Methods for enhancing and/or preserving activity of an enzyme in a liquid reaction mixture at a temperature of 30-100°C, and for refolding a protein are also disclosed.

## Description

### FIELD OF THE INVENTION

The present invention belongs to the technical field of additives for the improvement of enzymatic reactions of industrial interest. More specifically, the invention relates to chaperonins that may be used to protect the activity of high-temperature biotechnological and biomedical processes.

### BACKGROUND OF THE INVENTION

Enzymatic activities lose efficiency with the passage of reaction time, either because they are oxidized, because of structural instability in environments that are not optimal for the enzyme, such as high or low temperatures, pH far from neutrality, salinity or high ionic strength, etc., or because by their nature they have a half-life. However, it is well known that chaperonins protect enzymes from these effects by stabilising their structure or by assisting damaged units regain their native state. In particular, molecular chaperones of the Heat Shock Protein 60 family (HSP60), particularly of the GroEL type, have often been described as capable of assisting in the proper folding of other proteins. Often, these GroEL proteins are assisted by a second type of proteins, called co-chaperonins (GroES). Together, the GroEU GroEs protein complex of approximately 1 MDa that helps in the folding of other proteins that have undergone changes in their three-dimensional and chemical structure, such as exposure of hydrophobic residues to the environment that are normally found inside a protein. The GroEU GroES complex is ATP-dependent. The complex is capable of binding proteins with molecular weights no larger than 70 kDa. Many members of the HSP60 family possess enzymatic activities of industrial and biotechnological interest, and some have been described for their capacity for implementation in industrial processes. For example, US20070238158 (Method for enhancing enzyme activity at elevated temperature) and JP1998262693 (Regeneration of protein by using modified molecular chaperone) disclose the use of natural molecular chaperones such as GroEL from *Escherichia coli* to enhance enzymatic activity at high temperatures or for the regeneration of proteins.

In this context, there is a need to expand and improve the provision of a variety of molecular chaperones with suitable characteristics to be used in industrial settings in order to exploit their protective activity of high-temperature enzymatic processes of biotechnological and biomedical interest.

### DETAILED DESCRIPTION OF THE INVENTION

In view of the above need in this technical field, the inventors propose new versions of HSP60 molecular chaperones which have been obtained by means of Ancestral Sequence Reconstruction (ASR), wherein the amino acid sequence of the proposed proteins could have been the sequence of their equivalent versions from 2-4 billion years ago. These ancestral versions can have a much better protective effect of enzymatic activity at high temperature and could be used to enhance biotechnological reactions of industrial interest.

Thus, in a first aspect, the invention refers to an HSP60 protein, wherein the HSP60 protein has an amino acid sequence that has at least an 80% sequence identity with any one of SEQ ID NOS: 1-4.

As used herein, the term "HSP60", also known as chaperonins (Cpn), refers to a family of heat shock proteins originally sorted by their molecular mass, which is of approximately 60kDa. Chaperonins assist in the correct folding of denatured or *de novo* synthesised proteins into their native form. HSP60 generally belong to a large class of molecules that assist protein folding, called molecular chaperones. HSP60 chaperones are separated into three evolutionary classes, named Group I, Group II and Group III chaperonins:
- Group I chaperonins are monomeric structures arranged as two heptameric rings stacked back-to-back, found in bacteria and eukaryotic cells, and use a co-chaperonin (GroES). Their cellular distribution is mitochondrial/chloroplast.
- Group II chaperonins are typically multimeric structures (although some are known to be monomeric), consisting of two eight- (sometimes nine-) subunit-rings stacked back-to-back, found in archaea and eukaryotic cells, which do not require a co-chaperonin. Their cellular distribution is cytosolic.
- Group III chaperonins are structurally similar to Group II chaperonins. Group III was hypothesised to be a relic from an ancestral of both bacterial and archaeal chaperonins, although some studies have suggested a horizontal gene transfer (HGT) event from an ancestral archaeon.

The term "protein", as used herein, refers to a sequence of natural and/or synthetic amino acids, amino acid analogues or amino acid mimetics, having substantially similar or identical functionality, wherein the natural and/or synthetic amino acids are joined together by peptide bonds. Any analogues, derivatives, mimetics and the like may be incorporated such that the protein increases its stability, bioavailability, solubility, etc. "Analog", "derivative" and "mimetic" include molecules which sufficiently replicate the chemical structure of a peptidic structure and retain the functional properties of said peptidic structure. Approaches to designing peptide analogues, derivatives and mimetics are known in the art:
- A "derivative" (e.g., a protein or amino acid) includes forms in which one or more reaction groups on the compound have been derivatised with a substituent group. Examples of protein derivatives include proteins in which an amino acid side chain, the peptide backbone, or the amino- or carboxy-terminus has been derivatised.
- An "analogue" of a protein of the invention includes compounds which retain chemical structures as compared to a natural protein, which are necessary for functional activity, yet which also contain significantly different chemical structures. An example of an analogue of a naturally occurring protein is a protein which includes one or more non-naturally occurring amino acids.
- A "mimetic" of a compound includes compounds in which chemical structures of the compound necessary for functional activity have been replaced with other chemical structures which mimic the conformation of the compound (e.g., substitutions of the peptide backbone).

In the context of the instant invention, the term "amino acid" refers to naturally occurring and synthetic amino acids, as well as amino acid analogues and amino acid mimetics that function in a manner similar to the naturally occurring amino acids. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The term amino acid includes naturally occurring amino acids, uncommon natural amino acids, non-natural (synthetic) amino acids. The amino acids are preferably in the L configuration, but also D configuration, or mixtures of amino acids in the D and L configurations. The term "natural amino acids" comprises naturally occurring amino acids (Ala, Arg, Asn, Asp, Cys, Gin, Glu, Gly, His, lie, Leu, Lys, Met, Phe, Pro, Ser, Thr, Trp, Tyr, Val). As used herein the term "non-natural amino acid" refers to a carboxylic acid, or a derivative thereof, substituted at a position with an amine group and being structurally related to a natural amino acid. Examples of modified or uncommon amino acids are known in the art.

In particular embodiments of the invention, the HSP60 protein has an amino acid sequence that has at least an 80% sequence identity, at least an 85% sequence identity, at least an 88% sequence identity, at least a 90% sequence identity, at least a 91% sequence identity, at least a 92% sequence identity, at least a 93% sequence identity, at least a 94% sequence identity, at least a 95% sequence identity, at least a 96% sequence identity, at least a 97% sequence identity, at least a 98% sequence identity, at least a 99% sequence identity, at least a 99.5% sequence identity, at least a 99.9% sequence identity, or a 100% sequence identity, with any one of SEQ ID NOS: 1-4. In an embodiment, the sequence identity with any one of SEQ ID NOS: 1-4 is of at least 98%. In particular embodiments, the Hsp60 protein of the invention has an amino acid sequence as set forth in any one of SEQ ID NOS: 1-4.

In another aspect, the invention relates to a nucleotide sequence encoding for an Hsp60 protein according to the invention. The nucleotide sequence can be codon optimised for expression in specific cellular systems frequently used in the laboratory. Thus, in particular embodiments, the nucleotide sequence is codon optimised for expression in *E. coli* (Gram-negative bacteria), *Pseudomonas aeruginosa* (Gram-negative bacteria), *Salmonella spp* (Gram-negative bacteria), *Bacillus subtilis* (Gram-positive bacteria), *Streptomyces lividans* (Gram-positive bacteria); *Saccharomyces cerevisiae* (Yeast). In a specific embodiment, the nucleotide sequence is codon optimised for expression in *Escherichia coli.* In particular embodiments, the nucleotide sequence of the invention has a sequence as set forth in any one of SEQ ID NOS: 9-12.

In a further aspect, the invention relates to a composition comprising an Hsp60 protein according to the invention, and at least one enzyme selected from the group consisting of a protease, lipase, amylase, and cellulase. In the context of the instant invention, the term "protease" (also known as "peptidase" or "proteinase") refers to an enzyme capable of catalysing proteolysis, wherein the peptide bond is cleaved such that proteins are degraded into smaller polypeptides or individual amino acids. As used herein, the term "lipase" refers to a type of enzymes that catalyses the hydrolysis of fats. In the context of the invention, the term "amylase" refers to a hydrolytic enzyme that has the function of catalysing the hydrolysis reaction of 1-4 bonds between glucose units by digesting glycogen and starch to form fragments of glucose (dextrin, maltose) and free glucose. In the context of the invention, the term "cellulase" refers to a type of enzymes that can degrade the cellulose molecule into monosaccharides (simple sugars) such as β-glucose or into shorter polysaccharides and oligosaccharides. In another aspect, the invention relates to the use as a detergent of a composition comprising an Hsp60 protein according to the invention, and at least one enzyme selected from the group consisting of a protease, lipase, amylase, and cellulase. Thus, the Hsp60 proteins according to the present invention can be used as an additive component of compositions for washing clothes, or for cleaning surgical equipment.

In a further aspect, the invention relates to a composition comprising an Hsp60 protein according to the invention, and a glucosidase. In the context of the invention, the term "glucosidase" refers to glycoside hydrolase enzymes which are generally categorised under the EC number 3.2.1. In particular embodiments of the invention, the glucosidase can be selected from the group consisting of α-amylase (EC 3.2.1.1), β-amylase (EC 3.2.1.2), γ-amylase (EC 3.2.1.3), cellulase (EC 3.2.1.4), sucrase-isomaltase (EC 3.2.1.10), mannosyl-oligosaccharide glucosidase (EC 3.2.1.106), acid α-glucosidase (EC 3.2.1.20), beta-glucosidase (EC 3.2.1.21), lactase (EC 3.2.1.23), debranching enzyme (EC 3.2.1.33), and pullulanase (EC 3.2.1.41), among others. In another aspect, the invention relates to the use as a juice clarifying agent of a composition comprising an Hsp60 protein according to the invention, and a glucosidase.

In yet another aspect, the invention relates to a method for enhancing and/or preserving activity of an enzyme in a liquid reaction mixture at a temperature of 30-100°C, wherein the method comprises the step of adding to the liquid reaction mixture an HSP60 protein according to the invention. In the context of the invention, said "enhancing activity" is indicated by the presence of a rate of activity for said enzyme that is higher than the activity measured at the same temperature for the same enzyme in the absence of the HSP60 protein of the invention. Thus, data can be normalised to the activity of the enzyme alone (i.e., without the HSP60 protein) at that same temperature (100% activity). Once this reference value is established, the level of enhanced activity can be compared with this reference value, and thus be assigned a level of "enhanced". For example, an increase in activity levels above the reference value of at least 1.1-fold, 1.2-fold, 1.3-fold, 1.4-fold, 1.5-fold, 2-fold, 3-fold, 4-fold, 5-fold, or even more compared to the reference value is considered as an "enhanced" level of activity. In the context of the invention, said "preserving activity" is indicated by the presence of activity measured at a temperature at which said enzyme would normally be heat-inactivated wherein the reaction is conducted at a temperature of 30-100°C, and wherein the activity of the enzyme is measured, and a substrate is present at a temperature of 30-100°C. Data can be normalised to the activity of the enzyme alone (i.e., without the HSP60 protein) before it is subjected to temperature stress (100% activity). Once this reference value is established, the level of preserved activity can be compared with this reference value, and thus be assigned a level of "preserved". For example, activity levels compared to the reference value of at least 50%, at least 60%, at least 70%, at least 80%, at least 85%, at least 90%, at least 95%, at least 98%, at least 99% or of 100% is considered as a "preserved" level of activity. These two effects, "enhancing activity" and "preserving activity" are not mutually exclusive. Thus, it is possible in the context of the invention that enzymatic activity may be measured at a temperature at which said enzyme would normally be heat-inactivated and said activity being higher than the activity that said enzyme would show at a temperature at which said enzyme would normally not be inactivated. In particular embodiments of the invention, the reaction is conducted at a temperature of 30-100°C, 35-90°C, 40-80°C, 45-70°C, or 50-60°C. In particular embodiments of the invention, the reaction is conducted at a temperature of 30°C, 35°C, 40°C, 45°C, 50°C, 55°C, 60°C, 65°C, 70°C, 75°C, 80°C, 85°C, 90°C, 95°C or 100°C.

In a final aspect, the invention relates to a method of refolding a protein comprising the step of reacting a protein to be refolded with an HSP60 protein according to the invention. Preferred proteins to be refolded in the presence of the HSP60 protein disclosed herein with the method of the invention are DNA-polymerases, RNA-polymerase, agarases, chitosanases, chitinases, kinases, phosphatases, esterases, and the like. In a particular embodiment of the invention, the HSP60 protein is tagged with an hexahistidine-tag and bound to a Ni⁺ chelating agent (for example, nickel nitrilotriacetic acid (Ni-NTA)), allowing the refolding reaction to be performed using a Ni-NTA agarose column or the like. The advantage of such a setup is that the HSP60 protein can be repeatedly reused in the refolding reaction of the protein of interest.

As will be shown in more detail in the following examples, the selected ancestral HSP60 proteins show particular advantages in terms of the protective capacity of enzymatic activity at high temperatures (in the context of the invention, a temperature is considered a high temperature if it is a temperature with the range 35-100°C, e.g. 48°C):
- The HSP60 protein of SEQ ID NO.: 1 forms a single ring of 7 subunits and has a protective activity equivalent to a double-ring protein, which means that less protein content needs to be added to formulations.
- The activity of HSP60 protein of SEQ ID NO.: 3 is ATP-independent.
- Some have a 100 to 200% activity-protection effect, when compared to current equivalents.
- None of the ancestral HSP60 proteins need a co-chaperonin to confer their protective activity.

All the terms and embodiments described anywhere in this document are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

### EXAMPLES

The following invention is hereby described by way of the following examples, which are to be construed as merely illustrative and not limitative of the scope of the invention.

### Example 1: Rationale

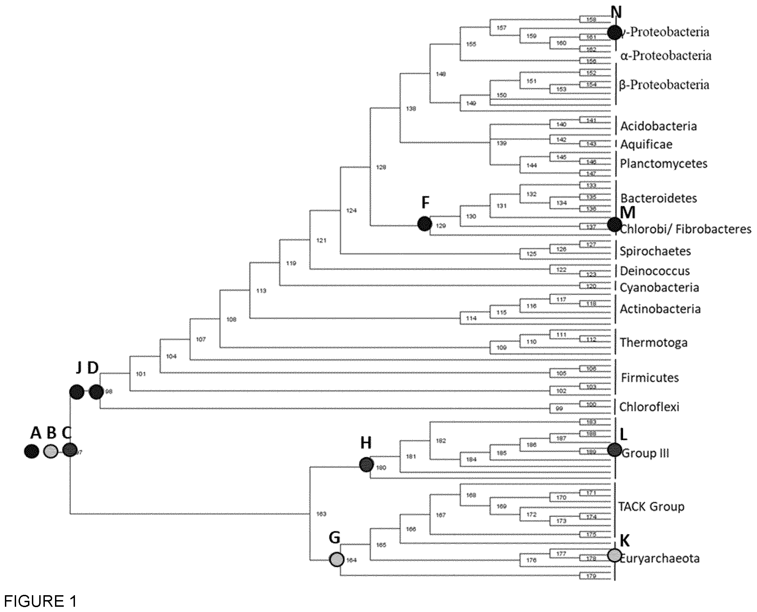

The objective of the invention was the *in silico* reconstruction and *in vitro* resurrection of ancestral Hsp60 proteins (i.e., chaperonins) by Ancestral Sequence Resurrection (ASR) strategies, wherein ASR identifies the most probable sequences at several ancestral nodes along the phylogenetic tree covering all life forms. Eight different sequences were initially selected following the step of *in silico* reconstruction, wherein the sequences belonged to the ancestral nodes indicated in figure 1. These sequences could correspond to the amino acid sequences of Hsp60 proteins of 2000-4000 million years ago.

The reconstruction of ancestral protein sequences using the ASR approach allows researchers to investigate the first proteins on Earth as well as the conditions under which they evolved. ASR, also known as paleogenetics, begins with a phylogeny of existing homologous sequences from which the probable ancestral sequences can be deduced. The ancestral sequences are then cloned into appropriate plasmids, depending on whether the characterisation is molecular or systems-level. The ancestral gene is expressed at the molecular level, and the ancestral protein is isolated for biochemical characterisation. The ancestral protein gene is inserted into the host genome, expressed, and the reconstructed paleophenotype is assessed *in vivo* on an organismal scale in the systems-level study. To date, approximately 211 ancestral proteins and 89 crystallized structures have been described. Although the general structure of these proteins has been conserved throughout their evolutionary history, these studies demonstrate how minor differences in protein sequence can cause significant changes in conformational dynamics, which may account for the differences in thermostability, catalytic efficiency, and substrate specificity reported for the reconstructions of the present invention.

### Example 2: in silico reconstruction

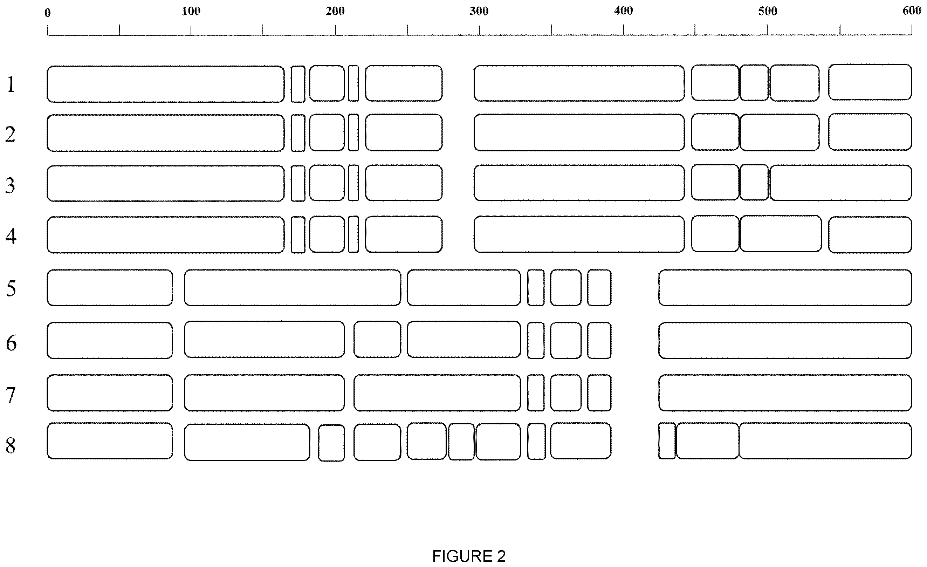

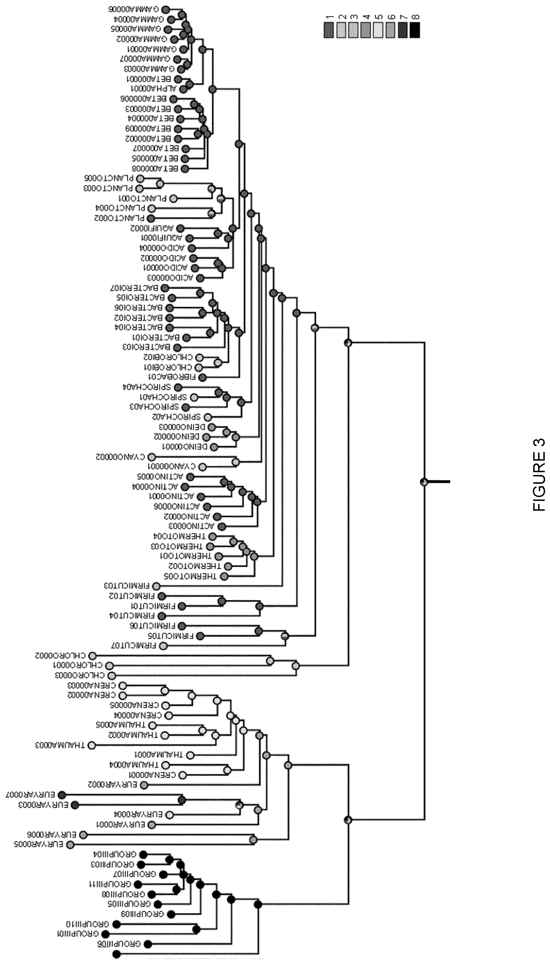

The step of *in silico* reconstruction (i.e., the first stage of ASR) was performed according to the following steps:
1. Selection of extant sequences: The chaperonin sequence from *E. coli* (UniProt Ref. No. P0A6F5) was used as a query on the KEGG (Kyoto Encyclopedia of Genes and Genomes) blast search server (https://www.genome.jp/tools/blast). Results were filtered by taxonomy, with a similarity threshold (10-6). The resulting FASTA format sequences were downloaded, and CD-HIT (Cluster Database at High Identity with Tolerance) was used to remove redundant sequences from the database using an identity threshold of 70% (http://weizhong-lab.ucsd.edu/cdhit suite). The *E. coli* sequence (UniProt Ref. No. P0A6F5) was used as a query on the STRING database (https://string-db.org/) using the GENE COOCCURRENCE function, in order to check its homologs phylogeny. When reconstructing, the tree should be similar to the STRING phylogeny.
2. Creation of a multiple alignment: Multiple sequence alignment (MSA) was performed with MAFFT (https://mafft.cbrc.jp/alignment/server). The model automatically chosen was L-INS-i. This model is used with small-scale alignments (N < ~200, L < -10 000). It is recommended in alignments with gaps and uses local alignment for scoring. This is helpful since three main sequence architectures were explored and a global alignment scoring might not be recommended in this context. The alignment was manually curated to remove abnormal gaps. Consensus sequence logo was generated from the alignment using WebLogo (https://weblogo.berkeley.edu/logo.cgi).
3. Choosing a substitution model of evolution: With the amino acid alignment, ProtTest was used to estimate a model of amino acid substitutions. The model chosen, according to the Akaike information criterion (AIC), was the LG model (Le and Gascuel 2008), with gamma distribution and a proportion of invariable sites (LG+I+G model, alpha (+I+G): 1.40).
4. Computing a phylogenetic tree: The phylogenetic history of 96 chaperonin sequences was reconstructed using the Bayesian method, implemented in MrBayes 3.2.7. The analysis used two independent Markov-chain Monte Carlo runs, with two chains each, performed over 800,000 generations until convergence was obtained (standard deviation of split frequencies = 0.01). Trees were sampled every 100 generations (>mcmcp samplefreq=100). Each run produced 8501 trees, from each 6376 were included, totalling 12,752 trees from which a consensus tree with high probabilities at the nodes was obtained. The LG amino acid substitution model was chosen based on ProtTest with gamma-distribution variation (>prset aamodelpr=fixed(lg)) and estimation of a proportion of invariable sites (>Iset rates=invgamma).
5. Computing ancestral sequences: Marginal reconstruction of ancestral sequences was done using PAML 4.9, with the LG substitution model with gamma-distributed rates across sites, using the phylogenetic tree obtained from MrBayes, rooted on chaperonin groups II and III, according to previous research, and the MSA obtained from MAFFT and manually curated. The most probable sequences at each ancestral node were selected. Homologous sequences were searched against the NCBI nr-Database, and the results are shown in table 1, herein below.
6. Identifying the most parsimony state at each ancestral node: Chaperonins sequences exhibit varied architectures inter- and intra-groups. From the alignment, eight different sequence "architectures" were defined (figure 2), in bacteria (architectures 1 to 4), archaea (architectures 5 to 7) and group III (architecture 8). The program Mesquite 3.61 was used to trace the character history of these indels across the phylogenetic tree (figure 3), using the parsimony method for the ancestral state reconstruction. The ancestral sequences were modified manually to match the closest modern sequence as determined by branch length, and according to the most parsimonious state at each node. Both methods were in accordance with each other.

**Table 1: Sequence similarity between ancestral and modern chaperonins.**

| **ASR sequence** | **Best hit (nr-NCBI data base)** | |
|---|---|---|
| | **% similarity** | **Taxon** |
| Sequence D Group I LCA | 57 | *Calorimonas adulescens* (WP_149544361.1) |
| | 59 | *Thermoanaerobacter* (WP_003866816.1) |
| | 60 | *Alicyclobacillus sendaiensis* (WP_062305661.1) |
| Sequence F Group I-FCB LCA | 81 | *Rhodothermaceae bacterium* (GIV58090.1) |
| | 81 | *Chloroherpetonaceae bacterium* (MCS6988128.1) |
| | 81 | *Candidatus Kapabacteria bacterium* (MCS6807187.1) |
| Sequence G Group II LCA | 62 | *Nanoarchaeota archaeon* (RLG17200.1) |
| | 63 | *Archaeoglobus profundus* (WP_012939672.1) |
| | 64 | *Thermococcus alcaliphilus* (WP_252742870.1) |
| Sequence H Group III LCA | 67 | *Thermosinus carboxydivorans* (WP_007289467.1) |
| | 67 | *Clostridia bacterium* (MDA8212682.1) |
| | 67 | *Desulfotomaculum nigrificans* (WP_003542311.1) |

The step of *in silico* reconstruction (i.e., the first stage of ASR) resulted in the selection of eight different sequences (nodes A, B, C, D, F, G, H and J in figure 1). In particular, nodes corresponding to the last common ancestor (LCA) of Group I (node D), Group I-FCB (node F), Group II (node G) and Group III (node H) were selected for resurrection. Additionally, four modern sequences were chosen for comparison: sequence N (*Escherichia coli* UNIPROT ID: P0A6F5, Group I model), sequence M (*Chlorobaculum tepidum* UNIPROT ID: Q8KF02, Group I-FCB model), sequence K (*Thermococcus strain* KS-1 alpha subunit UNIPROT ID: P61112, Group II model), and sequence L (*Carboxydothermus hydrogenoformans* UNIPROT ID: Q3AF10, Group III model). The selected proteins are the current counterparts of the revived proteins with known 3D structures. In terms of sequence identity, tables 2-4 shows a comparison of identity percentages between the sequences (AN) evaluated by the inventors, wherein the sequences have been organised according to Group I, Group II, or Group III Hsp60 classification.

**Table 2: Matrix of identity percentages between evaluated sequences in group I according to Hsp60 classification (sequences A, J, D, F, N and M). Sequences highlighted in italics could not be successfully expressed.**

| **Group I** | *A* | *J* | **D** | **F** | **N** | **M** |
|---|---|---|---|---|---|---|
| *A* | 100,00 | 71,82 | 73,26 | 51,33 | 43,40 | 44,53 |
| *J* | 71,82 | 100,00 | 96,68 | 60,82 | 53,02 | 53,22 |
| **D** | 73,26 | 96,68 | 100,00 | 60,49 | 52,17 | 53,67 |
| **F** | 51,33 | 60,82 | 60,49 | 100,00 | 70,61 | 77,51 |
| **N** | 43,40 | 53,02 | 52,17 | 70,61 | 100,00 | 65,81 |
| **M** | 44,53 | 53,22 | 53,67 | 77,51 | 65,81 | 100,00 |

**Table 3: Matrix of identity percentages between evaluated sequences in group II according to Hsp60 classification (sequences B, G and K). Sequences highlighted in italics could not be successfully expressed.**

| **Group II** | *B* | **G** | **K** |
|---|---|---|---|
| *B* | 100,00 | 65,45 | 44,94 |
| **G** | 65,45 | 100,00 | 60,57 |
| **K** | 44,94 | 60,57 | 100,00 |

**Table 4: Matrix of identity percentages between evaluated sequences in group III according to Hsp60 classification (sequences C, H and L). Sequences highlighted in italics could not be successfully expressed.**

| **Group III** | *C* | **H** | **L** |
|---|---|---|---|
| *C* | 100,00 | 59,07 | 38,73 |
| **H** | 59,07 | 100,00 | 56,12 |
| **L** | 38,73 | 56,12 | 100,00 |

### Example 3: in vitro resurrection

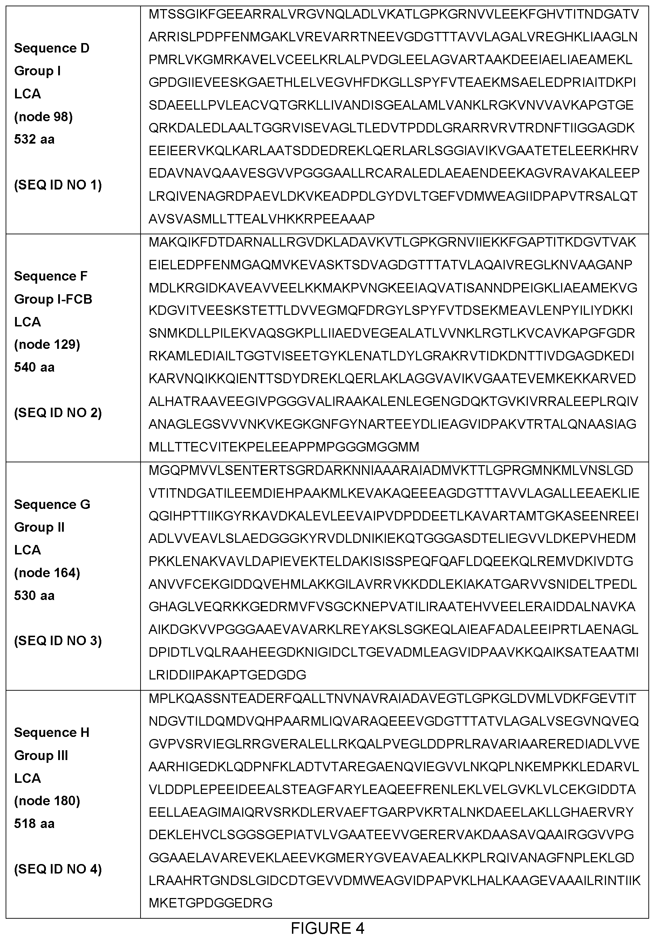

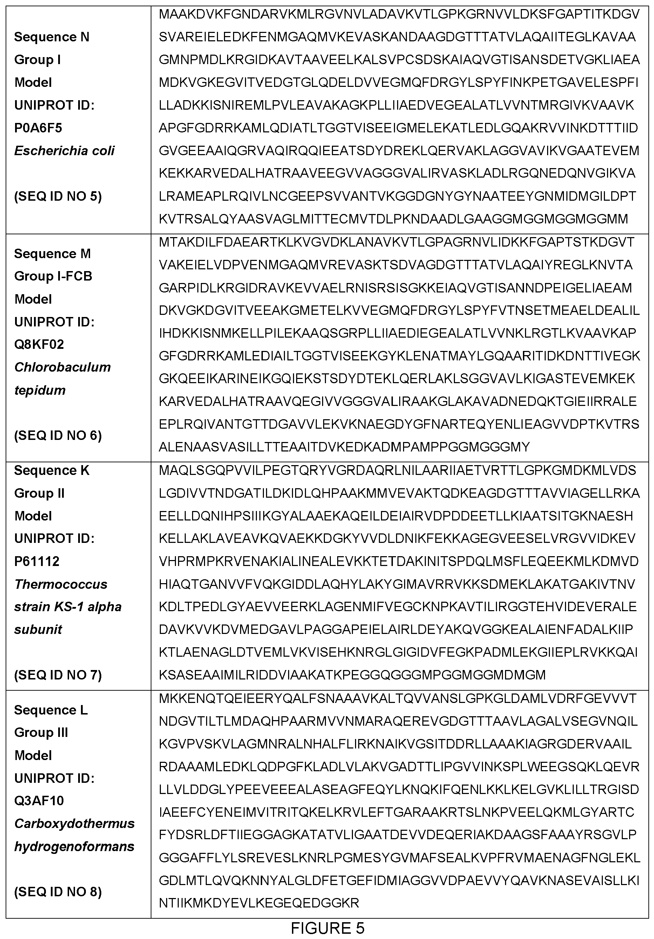

Of the eight different sequences that were initially selected (nodes A, B, C, D, F, G, H and J in figure 1), only four could be successfully expressed. Group I proteins A and J could not be expressed, despite having a sequence identity of 97% with ASR protein D, and more than 50% with modern proteins (N and M). In group II, sequence B could not be expressed, even though it was more similar to ASR protein G, than protein G was to modern protein K. Similarly, in group III, ASR protein C could not be expressed, despite having 59% sequence identity with ASR protein H, which is 56% identical to modern protein L. In these regards, the likelihood that any ASR-identified sequence produces a viable and/or functional protein decreases in earlier nodes. In any case, it should be noted that producing viable proteins going back as far as 3000-4000 million years is remarkable. The exact sequences of the four ancestral proteins that could be successfully expressed in the experimental work carried out by the inventors are indicated in figure 4, and the four modern sequences that were chosen for comparison are shown in figure 5.

In order to produce viable proteins out of the sequences identified in the *in silico* reconstruction stage, the nucleotide sequences corresponding to the above amino acid sequences were codon optimised for expression in an *E. coli* system and they were cloned into a pET-30a(+) plasmid (GenScript) with kanamycin resistance and a C-TEV cleavage sequence, followed by an hexahistidine-tag. The hexahistidine-tag binds specifically to nickel nitrilotriacetic acid (Ni-NTA) allowing the isolation and purification of the protein using a Ni-NTA agarose column. The TEV sequence can be used to remove the hexahistidine-tag after digestion with TEV protease. Thus, this method allows for the separation of the His-tagged Hsp-60 proteins in the cell lysates by IMAC (Immobilized Metal Ion Affinity Chromatography).

Specifically, *E. coli* strains were transformed with the protein coding plasmids (100 ng) and plated on LB agar with 1 mM kanamycin, overnight at 37°C. Protein expression was induced either by addition of IPTG to LB medium, or by autoinduction using the LB Broth Autoinducible with Trace Element (Condalab). Table 5 shows the protein overexpression settings that were employed.
- **IPTG Induction.** The grown colonies were used to inoculate a pre-culture of 100 ml of LB with 1mM kanamycin and incubated overnight at 37°C, after which they were used to inoculate larger cultures of 1-2 I of LB with 1 mM kanamycin at an OD600 = 0.1 (e.g. if cells grow O.N. to OD₆₀₀ ~3, we diluted 30X in LB medium, which means 25 ml of O.N. culture was added to 900 ml LB). Protein expression was induced with addition of 1 mM IPTG at the exponential growth phase (OD₆₀₀ = 0.5, note that cells duplicate every 30 minutes), following an incubation at 37°C for 4h.
- **Autoinduction.** Alternatively, cells were grown in LB Broth Autoinducible with Trace Element (Condalab) overnight at 28°C or 37°C. The cells were then harvested at 3.890 x g, or 5000 rpm in a Hitachi centrifuge with the R15A rotor, for 15 min at 4 °C and stored at -20°C.

**Table 5. Optimized conditions for overexpression of recombinant proteins.**

| **Plasmid** | **Sequence** | **Host** | **Induction** | **Temp.** | **Time** |
|---|---|---|---|---|---|
| D | LCA Group I | BL21DE3 | 1 mM IPTG | 37°C | 4h |
| F | LCA Group I-FCB | BL21DE3 | 1 mM IPTG | 37°C | 4h |
| G | LCA Group II | BL21DE3 | 1 mM IPTG | 37°C | 4h |
| H | LCA Group III | BL21DE3 | 1 mM IPTG | 37°C | 4h |
| K | P61112 | C41DE3 | Autoinduction | 28°C | overnight |
| L | Q3AF10 | BL21DE3 | Autoinduction | 28°C | overnight |
| M | Q8KF02 | BL21DE3 | Autoinduction | 37°C | overnight |
| N | P0A6F5 | BL21DE3 | Autoinduction | 37°C | overnight |

After thawing, the cell pellet was resuspended in nickel nitrilotriacetic acid (Ni-NTA) binding buffer (NiBB) (20 mM 4-(2-hydroxyethyl)-1-piperazineethanesulfonic acid (HEPES) pH 7.4, 20 mM imidazole, 500 mM KCI, 1 mM DTT and 15% glycerol (v/v)) supplemented with a protease inhibitor cocktail (Roche). The resuspended solution was sonicated three times for 1 min each time (cycles of 10 s *on,* 50 s off), with an amplitude of 50% in a Digital Sonifier 250 (Branson), and centrifuged at 35.000 - 40.000 rpm in a Beckman Coulter ultracentrifuge with a Type 50.2 Ti rotor, for 40 min at 4°C.

The supernatant was filtered (0.45 µm filters and a 10 ml syringe) and loaded onto a Ni-NTA agarose column (HisTrap FF 5 ml, GE Healthcare) using a fast protein liquid chromatography (FPLC) apparatus (GE Healtchare). Before usage, the buffers NiBB and NiEB were filtered (0.22 µm) with a vacuum pump. After the sample was loaded, the column was washed with 5 column volumes (cv) of NiBB and then eluted with Ni-NTA elution buffer (NiEB) (same as NiBB with 500 mM imidazole) using a gradient of 50% over 15 minutes. The fractions (2 ml) were collected and analyzed by SDS-PAGE (sodium dodecyl sulphate polyacrylamide gel electrophoresis), and those fractions containing the corresponding protein were pooled and concentrated by centrifugation (3.890 x g, or 5000 rpm in a Hitachi centrifuge with the R15A rotor, 4°C) using 50 kDa or 100 kDa cut-off Amicon Ultra 15 filters (Millipore).

The concentrated fractions containing the protein of interest were loaded onto size exclusion chromatography (SEC) Superose^{®} 6 Increase 10/300 GL (GE Healthcare) columns, previously equilibrated in SEC buffer (SECB) (20 mM HEPES pH 7.4, 150 mM KCI, 10 % glycerol (v/v)). Proteins were eluted in 1 column volume and fractions (0.5 ml) were analyzed by SDS-PAGE and by negative staining electron microscopy. To induce complex formation, when necessary, 25 mM MgCl2 and 5 mM ATP were added to SECB, meaning that oligomerisation was promoted under optimised conditions for each sequence (for example, sequence D required the addition of MgCl₂ and ATP). Some cases required vitrification of the most significant fraction for Cryo-EM observations. Fractions with proteins forming the typical barrel-shaped multimeric complex were snap frozen in liquid nitrogen and stored at -20°C.

The particular procedure and results in respect of each of the ancestral sequences (group I, group I-FCB, group II and group III) is detailed as follows.

### 3.1. Group I chaperonins last common ancestor (LCA).

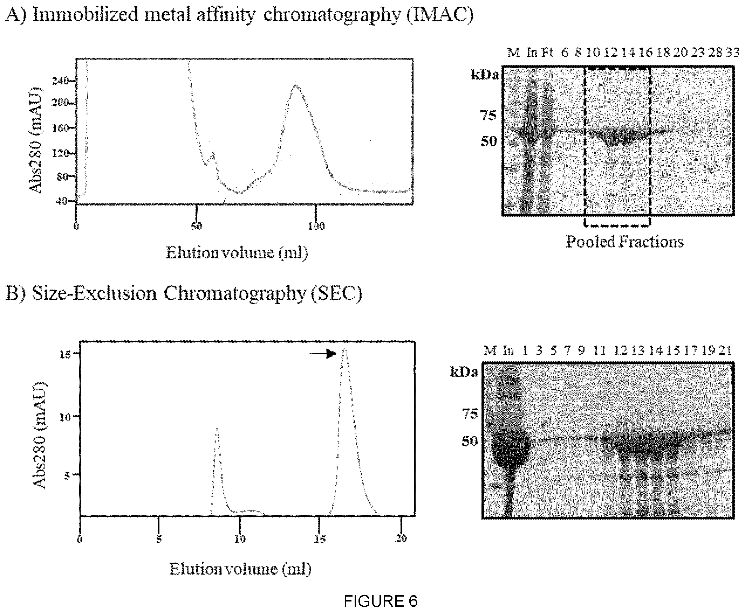

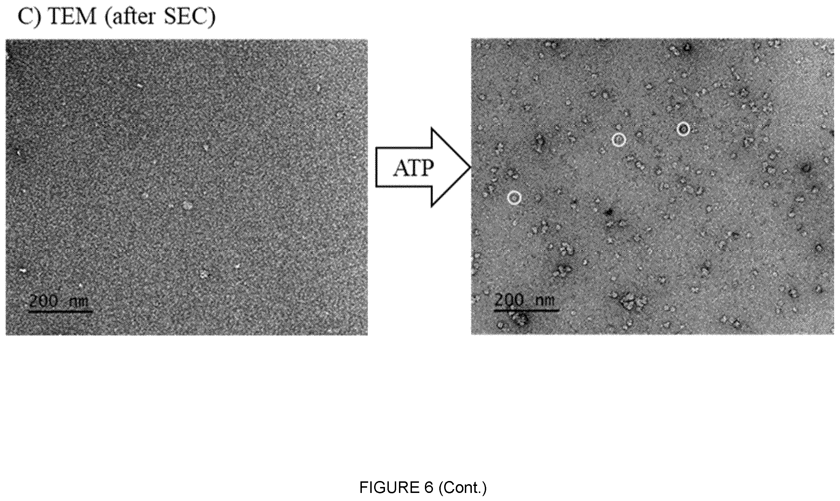

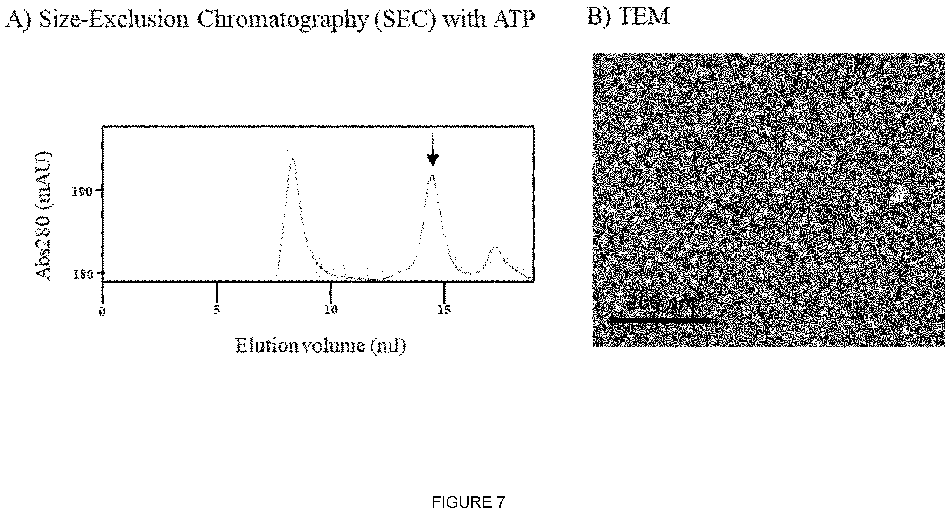

*E. coli* BL21 cells were transformed with a plasmid carrying the sequence of the LCA of Group I chaperonins (Plasmid D), showing 57-60% sequence similarity to modern thermophilic organisms (Table 1). Cells were grown for 4h at 37°C, after IPTG induction, and harvested by centrifugation. The his-tagged proteins were separated from the cell lysate by IMAC (Figure 6A). After SDS-PAGE analysis, IMAC fractions 10-16 were pooled and further purified by SEC (Figure 6B). TEM analysis of the fractions obtained by SEC showed that the chaperonin subunits were not assembling in the typical multimeric structure (Figure 6C). In an attempt to promote oligomerization, the incubation of SEC fraction 13, corresponding to the highest peak in the SEC chromatogram, was tested with 25 mM MgCl₂ and 5 mM ATP at 37°C for 1h. Some oligomers were achieved (Figure 6C, circles). Results showed that addition of MgCl₂ and ATP promoted the oligomerization of ancestral Group I chaperonins. In order to recover oligomers, SEC using an ATP-containing buffer (Figure 7A) separated pooled fractions from a second IMAC purification. This allowed to recover the oligomerized chaperonins, as observed by TEM (Figure 7B). Fractions corresponding to the arrow pointed peak in Figure 7A were pooled and structure stability was evaluated by TEM immediately after purification (Figure 7B) and after 24h at 4°C (not shown). Integrity was observed and the pool was used for vitrification. The 3D structure reconstructed by Cryo-Em showed a single-ring 7-mer assembly, typical of single-ring Group I chaperonins (not shown). From the top view, there appears to be a substrate in the cavity of the chaperonin.

### 3.2. Group I-FCB chaperonins last common ancestor (LCA).

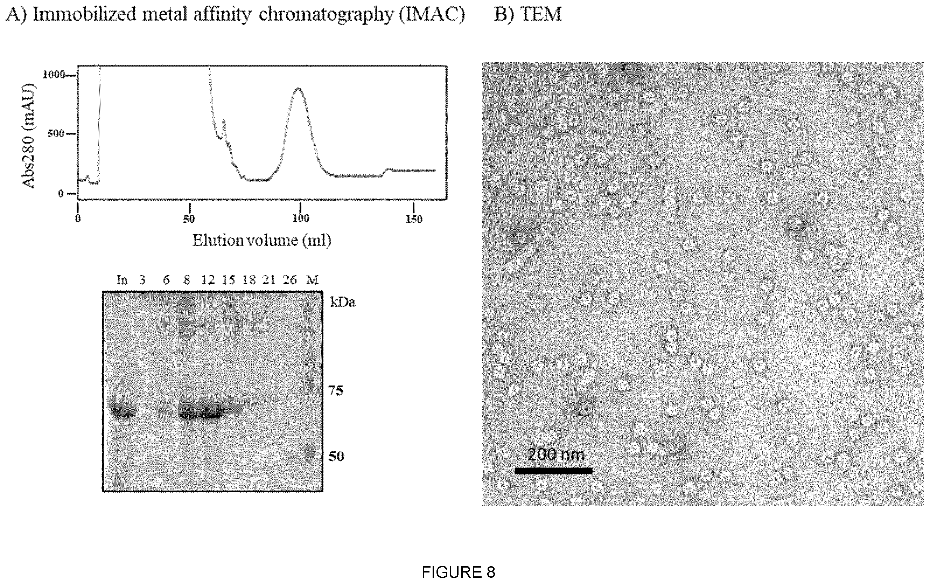

*E. coli* BL21 cells were transformed with a plasmid carrying the sequence of the LCA of FCB Group chaperonins (Plasmid F), showing 81% sequence similarity to modern thermophilic organisms (Table 1). Cells were grown for 4h at 37°C, after IPTG induction, and harvested by centrifugation. The his-tagged proteins were separated from the cell lysate by IMAC (Figure 8A). TEM analysis showed that fraction 12 was pure enough for vitrification (Figure 8B) and was also used for antibody production. The 3D structure reconstructed by Cryo-Em showed a double-ring 14-mer assembly, typical of Group I chaperonins, to which the atomic model predicted by AlphaFold was fitted (not shown) (https://alphafold.ebi.ac.uk/).

### 3.3. Group II (Archaeal) chaperonins last common ancestor (LCA).

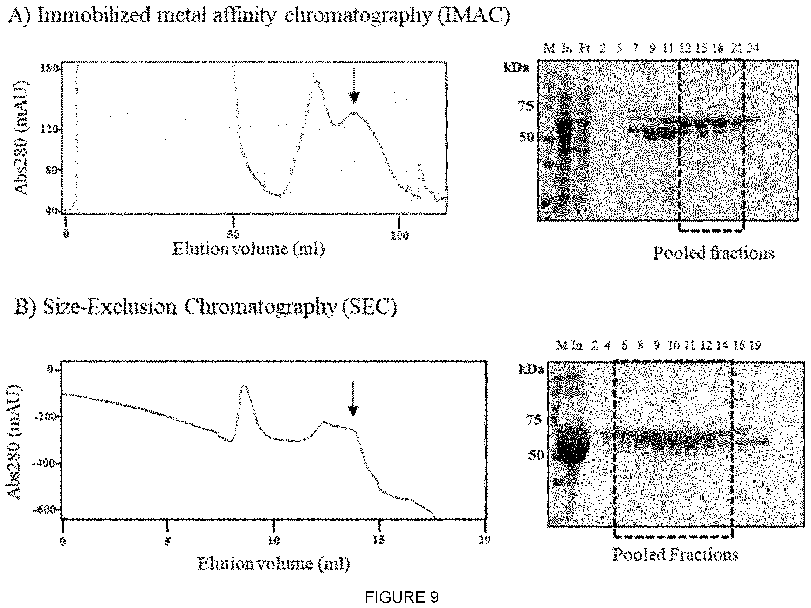

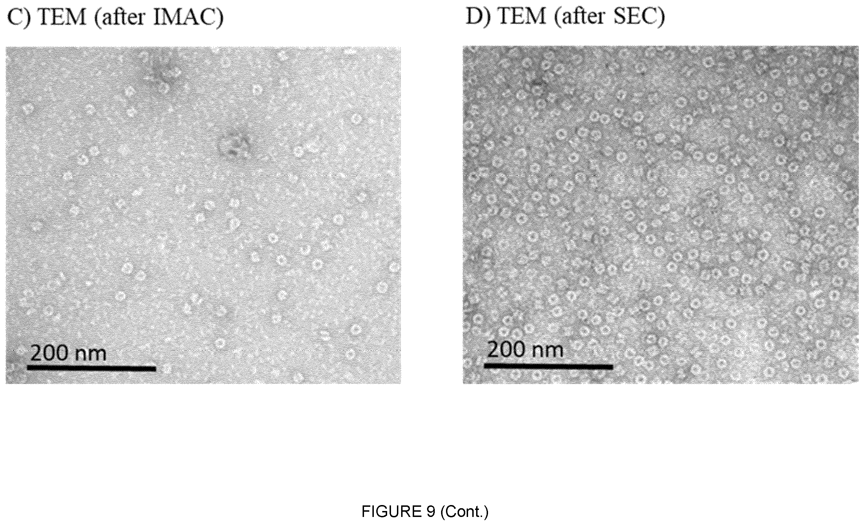

*E. coli* BL21 cells were transformed with a plasmid carrying the sequence of the LCA of Group II chaperonins (Plasmid G), showing 62-64% sequence similarity to modern thermophilic organisms (Table 1). The cells were grown for 4h at 37°C, after IPTG induction, and harvested by centrifugation. The his-tagged proteins were separated from the cell lysate by IMAC (Figure 9A) and analysed by SDS-PAGE and TEM (Figure 9C). After analysis, IMAC fractions 12-21 were pooled and separated by SEC (Figure 9B). After TEM analysis (Figure 9D), fraction 12 was vitrified and used for 3D Cryo-EM reconstruction. SEC fractions 6-14 were pooled and used for antibody production. The 2D structure classification of particles obtained by Cryo-Em showed a double-ring 16-mer assembly (not shown).

### 3.4. Group III chaperonins last common ancestor (LCA).

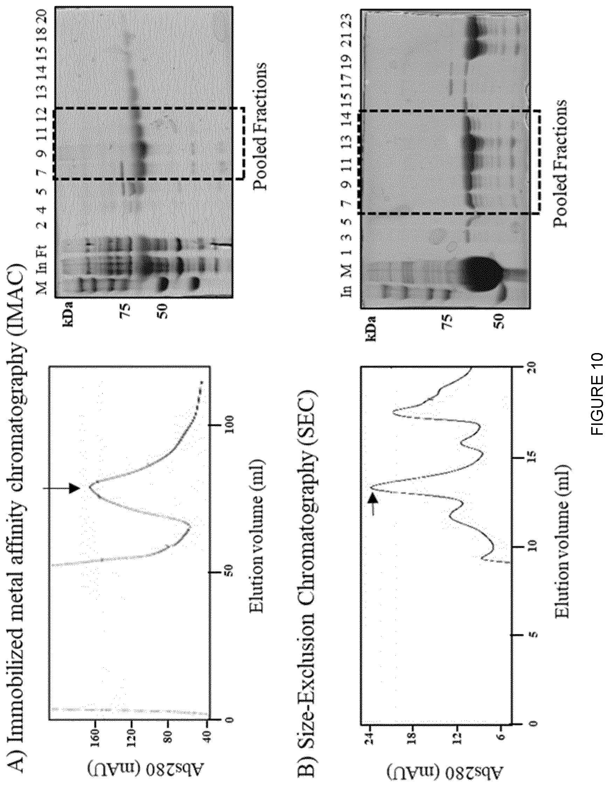

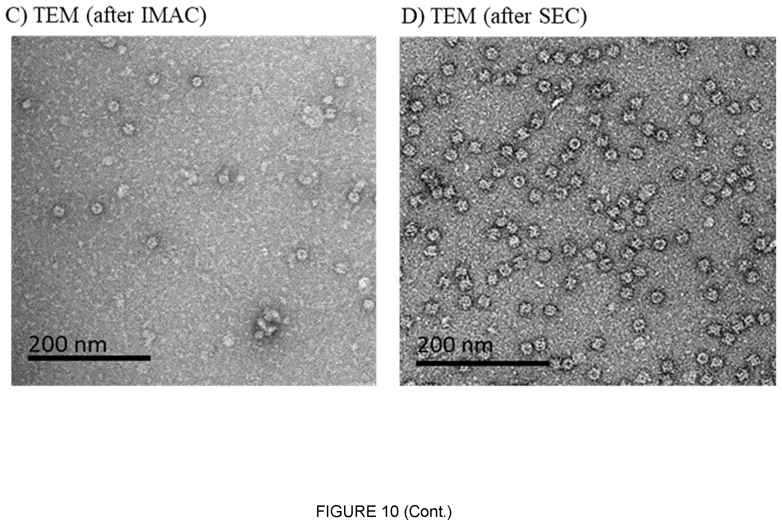

*E. coli* BL21 cells were transformed with a plasmid carrying the sequence of the LCA of Group II chaperonins (Plasmid H), showing 67% sequence identity to modern thermophilic and acid mine drainage organisms (Table 1). The cells were grown for 4h at 37°C, after IPTG induction, and harvested by centrifugation. The his-tagged proteins were separated from the cell lysate by IMAC (Figure 10A) and analysed by SDS-PAGE and TEM (Figure 10C). After SDS-PAGE analysis, IMAC fractions 7-12 were pooled and separated by SEC (Figure 10B). SEC fraction 11 (Figure 10C) was vitrified and used for 3D Cryo-EM reconstruction. SEC fractions 7-14 were pooled and used for antibody production. The 3D structure reconstructed by Cryo-Em (not shown) showed a double-ring 16-mer assembly as reported for *C*. *hydrogenoformans* Group III chaperonin.

### Example 4: Functional assays

### 4.1: ATPase activity

The ATPase activity of five Hsp60 proteins, one modern control protein (*Carboxydothermus hydrogenoformens,* a Group III model organism) and four ancestral chaperonins (Groups I, I-FCB, II, and III), was assessed between 30°C and 60°C, with increments of 10°C. The ATP hydrolysis rates of ancestral and *C*. *hydrogenoformans* chaperonins were calculated based on the theoretical molecular weight of its oligomer complex. The theoretical molecular weight of each subunit was calculated from the protein amino acid sequence using the ExPASy - ProtParam tool (https://web.expasy.org/protparam/). The molecular weight of each complex was calculated by multiplying the molecular height of each subunit to the number of subunits in each complex (Table 6).

**Table 6: Chaperonins used in the ATPase activity assay - molecular weight.**

| **Protein** | **MW subunit** | **Assembly** | **MW kDa** |
|---|---|---|---|
| D | 58 kDa | Single ring, 7-mer | 406 kDa |
| F | 58 kDa | Double ring, 14-mer | 814 kDa |
| G | 57 kDa | Double ring, 16-mer | 912 kDa |
| H | 56 kDa | Double ring, 16-mer | 896 kDa |
| L | 57 kDa | Double ring, 16-mer | 912 kDa |

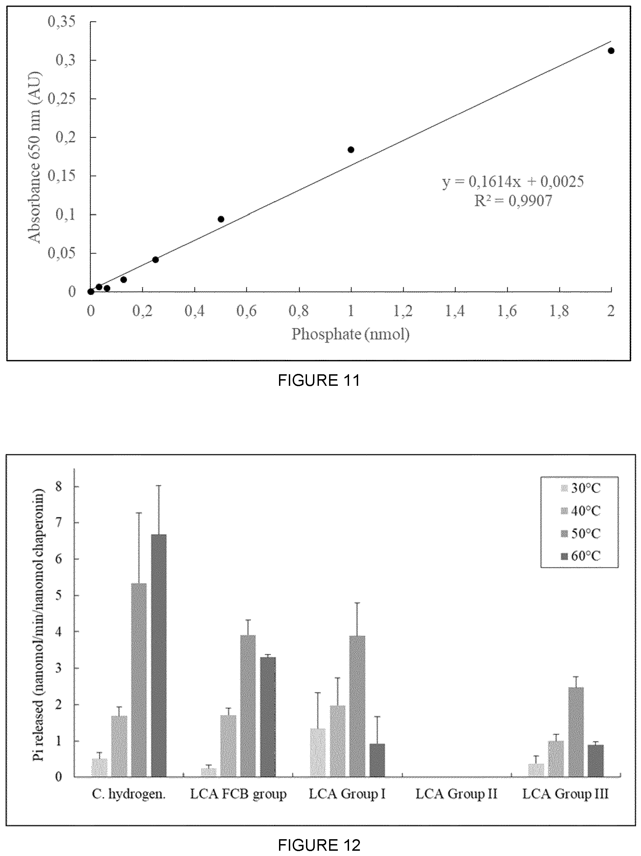

Chaperonins with concentrations of 0.35 µg/µl or 0.5 µg/µl, corresponding to molar concentrations ranging from 0.4 µM and 0.6 µM, were incubated in a 25 µl of buffer containing 50 mM HEPES pH 7.4, 150 mM KCI and 5 mM MgCl2, to which an equal volume the same buffer containing 0.5 mM ATP was added. Tests without chaperonin were used as a reference. Incubation was carried out on a thermoblock with gentle agitation (300 rpm) at a range of temperatures (30°C, 40°C, 50°C and 60°C). After 1h, each reaction was mixed with 100 µl of BIOMOL^{®} Green Reagent (Enzo Life Sciences, USA) in 96-well plates. This reagent forms a complex with the phosphate released during ATP hydrolysis. After 20 minutes at RT, absorbance at 650 nm was measured using a SpectraMax^{®} iD3 spectrophotometer. The amount of phosphate released was approximated using a function generated from a phosphate standard curve (Figure 11) with a series of dilutions ranging from 2 to 0 nmol phosphate (Pi), following the manufacturer's instructions.

Results showed that all but the Group II chaperonin showed ATPase activity at the tested conditions (Figure 12). Ancestral chaperonins showed a peak in ATPase activity at 50°C and a decrease at 60°C. The ATPase activity of the modern chaperonin from *C*. *hydrogenoformens* was higher at 60°C.

### 4.2: Chaperone activity

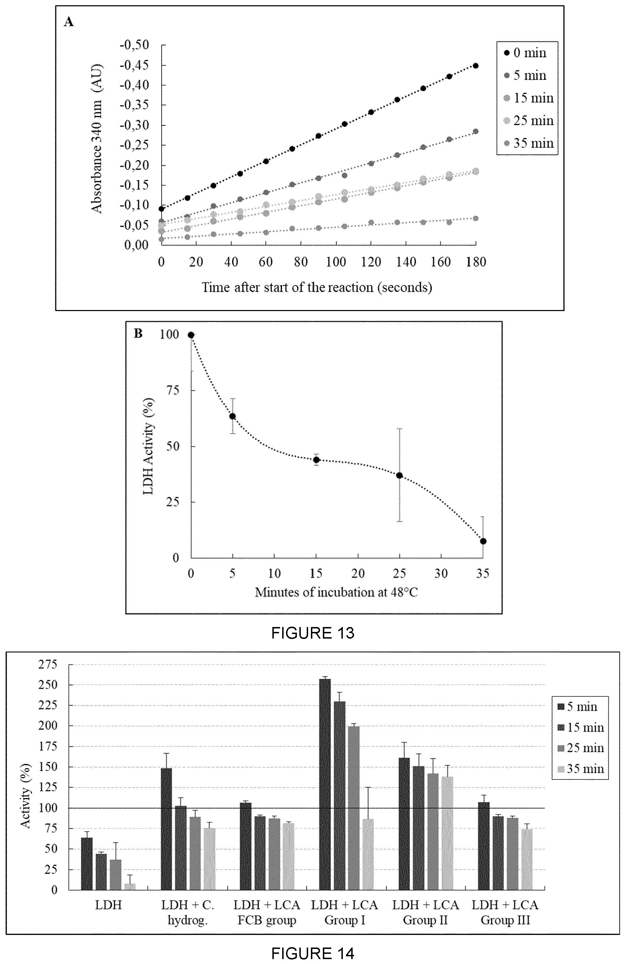

The enzyme lactate dehydrogenase (LDH) catalyses the synthesis of lactate and nicotinamide adenine dinucleotide (NAD+) from pyruvate, as well as its reduction to NADH. Since NADH absorbs light at 340 nm, it can be measured with a spectrophotometer giving an indication of the activity of the enzyme. It is known that the enzyme lactate dehydrogenase (LDH) loses 50% of its activity after 15 minutes at 48°C (Figure 13). After 35 minutes at 48°C, almost no LDH activity can be detected. Using this experimental readout, LDH protection from heat inactivation by ASR Hsp60 chaperonins was tested.

In general, all tested chaperonins, four ancestral (sequences D, F, G and H) and one modern *C*. *hydrogenoformans* chaperonin (sequence L), protected LDH from heat inactivation (Figure 14). Specific behaviours were recorded as follows:
- Sequence D (group I): In the presence of the LCA of Group I (1 µM), LDH activity increased to 250% in the first 5 minutes of incubation, descending to 200% activity after 15 minutes, only to descend to its normal (unstressed) activity after 35 minutes.
- Sequences G (group II): Activity of LDH remained stable around 125-150% activity during the 35 minutes when in the presence of the Group II LCA (0.5 µM).
- Sequences F and H (groups III and I-FCB): In the presence of the LCA of Groups III and I-FCB (both at 0.5 µM), there was a slight increase in LDH activity in the first 5 minutes of incubation, but this activity descended to approximately 75% after 35 minutes.
- Sequence L (control): LDH activity increased after 5 minutes of incubation with *C*. *hydrogenoformans* chaperonin (0.5 µM), but after 15 minutes LDH activity returned to normal, un-stressed values, with a slight descent to 75% activity after 35 minutes incubation.

### BRIEF DESCRIPTION OF THE FIGURES

- **Figure 1****:** Cladogram showing ancestral nodes selected for protein resurrection, wherein specific nodes (bold letters A, B, C, D, F, G, H and J) correspond to the last common ancestors (LCA) of Group I (A, D, J, F dots), Group II (B, G dots), and Group III (C, H dots) chaperonins selected for resurrection, and wherein four modern sequences (bold letters K, L, M and N) have been selected for comparison.
- **Figure 2****:** Protein sequence architectures of HSP60 chaperones.
- **Figure 3****:** Ancestral character reconstruction. Ancestral character reconstruction using Mesquite 3.61 showing the most parsimonious state at each ancestral node, using the parsimony method for the ancestral state reconstruction. The legend numbering represents the possible sequence architectures.
- **Figure 4****:** Exact sequences of four ancestral proteins that could be successfully expressed in the experimental work.
- **Figure 5****:** Exact sequences of four modern sequences that were chosen for comparison with ancestral proteins.
- **Figure 6****:** Purification of LCA of Group I chaperonins. Chromatography results, SDS-PAGE analysis and TEM micrographs are shown for the purification steps with (A) Ni-NTA and (B) Superose^{®} 6 Increase 10/300 GL (GE Healthcare) columns. TEM micrographs are shown for SEC fraction 13 (C) after SEC purification (left) and after addition of ATP (right). Circles enclose frontal views of the ring structures.
- **Figure 7****:** Purification of LCA Group I chaperonins with ATP. Chromatography results and TEM micrograph are shown for the purification step with Superose^{®} 6 Increase 10/300 GL (GE Healthcare) column. The faction corresponding to the arrow was pooled, observed by TEM (B) and vitrified after 24h, once chaperonins' integrity was checked once again by TEM.
- **Figure 8****:** Purification of LCA FCB Group I chaperonins. Chromatography results (A), SDS-PAGE analysis and Transmission electron microscopy (TEM) micrograph (B) is shown for the purification step with Ni-NTA column. After analyzing the fractions by TEM, fraction 12, corresponding to the arrow in (A) and the micrograph in (B), was vitrified. Fractions were snap frozen in liquid nitrogen and stored at -20°C.
- **Figure 9****:** Purification of Group II LCA chaperonins. Chromatography results, SDS-PAGE analysis and TEM micrographs are shown for the purification steps with (A, C) Ni-NTA and (B, D) Superose^{®} 6 Increase 10/300 GL (GE Healthcare) columns. After analysing the fractions, those with the best-assembled and greater number of chaperonins were pooled, snap frozen in liquid nitrogen and stored at -20°C. Arrows in the chromatograms indicate the fractions observed by TEM.
- **Figure 10****:** Purification of Group III LCA chaperonins. Chromatography results, SDS-PAGE analysis and TEM micrographs are shown for the purification steps (A, C) Ni-NTA and (B, D) Superose^{®} 6 Increase 10/300 GL (GE Healthcare) columns. After analysing the fractions, those with the best-assembled and greater amount of chaperonin were pooled, snap frozen in liquid nitrogen and stored at -20°C. Arrows in the chromatograms indicate the fractions observed by TEM.
- **Figure 11****:** Standard phosphorus concentration curve. Example of a standard curve obtained with known phosphate concentration, for calculating the amount of phosphate released in a reaction (ATPase assay) using the BIOMOL^{®} Green Reagent (Enzo Life Sciences, USA) and a spectrophotometer.
- **Figure 12****:** ATPase activity of chaperonins as a function of temperature. Chaperonins were incubated in the presence of MgCl₂ (5 mM), KCI (150 mM) and ATP (0.25 mM), at the indicated temperatures.
- **Figure 13****:** Activity of lactate dehydrogenase under heat stress. The activity of LDH enzyme was recorded at 340 nm. The different data points on panel (A) represents the raw data readout from each time-point during the deactivation by high temperature (48°C). The lines represent the slopes calculated for the initial linear phase of each reaction. The observed activity of the enzyme drops as shown by the decreasing slopes as a function of time. (B) The deactivation of LDH enzyme is shown as percentage relative remaining activity. The data for each enzyme was normalized to the activity of the non-stressed enzyme. The data was plotted as mean and SD (n=2).
- **Figure 14****:** Hsp60 chaperonins protect lactate dehydrogenase from heat inactivation. The activity of the enzyme LDH (0.1 µM) was measured after temperature stress (48°C) in the presence of different chaperonins. Data was normalised to the activity of the enzyme alone before it was subjected to temperature stress (100% activity).

## Claims

1. An HSP60 protein, wherein the HSP60 protein has an amino acid sequence that has at least an 80% sequence identity with any one of SEQ ID NOS: 1-4.

2. The Hsp60 protein according to claim 1, wherein the Hsp60 protein has an amino acid sequence as set forth in any one of SEQ ID NOS: 1-4.

3. A nucleotide sequence encoding for an Hsp60 protein according to any one of claims 1 or 2.

4. The nucleotide sequence according to claim 3, wherein the nucleotide sequence has a sequence as set forth in any one of SEQ ID NOS: 9-12.

5. A composition comprising an Hsp60 protein according to any one of claims 1 or 2, and at least one enzyme selected from the group consisting of a protease, lipase, amylase, and cellulase.

6. Use of a composition according to claim 5, as a detergent.

7. A composition comprising an Hsp60 protein according to any one of claims 1 or 2, and a glucosidase.

8. Use of a composition according to claim 7, as a juice clarifying agent.

9. A method for enhancing and/or preserving activity of an enzyme in a liquid reaction mixture at a temperature of 30-100°C, wherein the method comprises the step of adding to the liquid reaction mixture an HSP60 protein according to any one of claims 1 or 2.

10. A method for refolding a protein comprising the step of reacting a protein to be refolded with an HSP60 protein according to any one of claims 1 or 2.
